# EUROPÄISCHE PATENTSCHRIFT

(11) **EP 4 161 628 B1**
(45) Veröffentlichungstag und Bekanntmachung des Hinweises auf die Patenterteilung: **13.08.2025**
(21) Anmeldenummer: 21728577.4
(22) Anmeldetag: 28.05.2021
(51) Int. Cl.: A61M 60/109, A61M 60/279, A61M 60/37

(54) **PERISTALTIKPUMPE FÜR EINE VORRICHTUNG ZUR EXTRAKORPORALEN BLUTBEHANDLUNG**
PERISTALTIC PUMP FOR A DEVICE FOR EXTRACORPOREAL BLOOD TREATMENT
POMPE PÉRISTALTIQUE POUR UN DISPOSITIF DE TRAITEMENT EXTRACORPOREL DU SANG

(30) Priorität: 03.06.2020 DE 102020206875
(43) Veröffentlichungstag der Anmeldung: 12.04.2023
(73) Patentinhaber: B. Braun Avitum AG, 34212 Melsungen (DE)
(72) Erfinder: HORSCHKE, Sebastian, 37236 Hessisch Lichtenau (DE); TEICHMANN, Oliver, 34576 Homberg (DE)
(74) Vertreter: Patentanwälte Ruff, Wilhelm, Beier, Dauster & Partner mbB
(86) Internationale Anmeldenummer: PCT/EP2021/064389
(87) Internationale Veröffentlichungsnummer: WO 2021/244977

(56) Entgegenhaltungen:
- EP-A1- 1 442 758
- EP-A1- 3 689 387
- EP-B1- 1 442 758
- WO-A1-2009/025686
- WO-A1-2019/065480
- DE-A1- 102007 058 533
- DE-A1- 102014 004 476
- US-A1- 2018 230 987

## Beschreibung

Die Erfindung betrifft eine Peristaltikpumpe für eine Vorrichtung zur extrakorporalen Blutbehandlung, aufweisend einen um eine Rotorachse angetrieben rotierbaren Rotor, und aufweisend ein Pumpengehäuse mit einer Aufnahmeaussparung, in welcher der Rotor aufgenommen ist, und welche eine bogenförmig um die Rotorachse erstreckte und radial von dem Rotor beabstandete Stützfläche aufweist, wobei die Stützfläche zum Abstützen einer radial zwischen dem Rotor und der Stützfläche in der Aufnahmeaussparung positionierbaren Fluidleitung vorgesehen ist, die zur Pumpförderung eines durch die Fluidleitung zu fördernden Fluids unter mechanischer Einwirkung des rotierenden Rotors abschnittsweise zwischen dem Rotor und der Stützfläche elastisch deformierbar ist, und wobei das Pumpengehäuse wenigstens einen Führungskanal aufweist, der zwischen einer Außenseite des Pumpengehäuses und der Aufnahmeaussparung durch eine Gehäusewandung des Pumpengehäuses erstreckt ist und zum Führen der Fluidleitung zwischen der Außenseite und der Aufnahmeaussparung vorgesehen ist.

Bekannte Vorrichtungen zur extrakorporalen Blutbehandlung, insbesondere Dialysegeräte, weisen eine Peristaltikpumpe zur Pumpförderung des zu behandelnden Bluts in einem extrakorporalen Blutkreislauf der Vorrichtung auf. Optional oder zusätzlich wird eine weitere Peristaltikpumpe zur Förderung von Dialysierflüssigkeit genutzt. Hierfür bekannte Peristaltikpumpen weisen üblicherweise einen Rotor, ein Pumpengehäuse und eine zwischen Rotor und Pumpengehäuse angeordnete elastisch deformierbare Fluidleitung auf. Der Rotor ist um eine Rotorachse angetrieben in einer Aufnahmeaussparung des Pumpengehäuses aufgenommen, die auch als Pumpenbett bezeichnet wird. Das Pumpenbett weist eine bogenförmig um die Rotorachse erstreckte Stützfläche für die Fluidleitung auf. Zur Pumpförderung des Bluts wird die Fluidleitung unter Einwirkung des rotierenden Rotors abschnittsweise gegen die Stützfläche gepresst und hierdurch elastisch zusammengequetscht. Der abgequetschte Abschnitt der Fluidleitung wandert gemeinsam mit dem Rotor um die Rotorachse, so dass das zu fördernde Blut durch die äußere Verformung der Fluidleitung durch diese hindurchgedrückt wird. Das Pumpengehäuse ist häufig einteilig gestaltet. Ein solch einteiliges Pumpengehäuse ist beispielsweise in der EP 3 281 653 A1 als Stand der Technik gewürdigt. Das bekannte Pumpengehäuse weist zwei zwischen einer Außenseite und der Aufnahmeaussparung gerade längserstreckte Führungskanäle für die Fluidleitung auf. Die Führungskanäle bilden einen Ein- und einen Auslass für die Fluidleitung. Um ein unerwünschtes manuelles Eingreifen in die Aufnahmeaussparung und damit den Arbeitsbereich des Rotors zu vermeiden, wird das bekannte Pumpengehäuse üblicherweise mit einem Deckel verschlossen. Sofern keine Fluidleitung in die Führungskanäle eingelegt ist, liegen deren Querschnitte frei.

Andere Pumpengehäuse sind aus DE102014004476A1 und US2018230987A1 bekannt.

Aufgabe der Erfindung ist es, eine Peristaltikpumpe der eingangs genannten Art bereitzustellen, die eine gegenüber dem Stand der Technik verbesserte Anwendungssicherheit bietet und gleichzeitig einen einfachen Aufbau aufweist.

Diese Aufgabe wird dadurch gelöst, dass das Pumpengehäuse einen durch eine wenigstens abschnittsweise längsgekrümmte Gestaltung des Führungskanals ausgebildeten Eingriffsschutz aufweist, wobei ein Krümmungsradius des Führungskanals derart auf eine Kanallänge und einen Kanaldurchmesser des Führungskanals abgestimmt ist, dass ein manuelles Eingreifen durch den Führungskanal in die Aufnahmeaussparung verhindert ist. Durch die erfindungsgemäße Lösung ist ein durch den wenigstens einen Führungskanal gerichtetes manuelles Eingreifen in die Aufnahmeaussparung selbst dann verhindert, wenn keine Fluidleitung in den Führungskanal eingelegt ist. Hierdurch kann die Anwendungssicherheit maßgeblich verbessert werden. Denn üblicherweise wird die Fluidleitung nach jeder Nutzung ersetzt, so dass der wenigstens eine Führungskanal wenigstens zeitweise freiliegt. In einem solchen Zustand kann es bei aus dem Stand der Technik bekannten Peristaltikpumpen zu einem manuellen Eingreifen durch den Führungskanal in die Aufnahmeaussparung und damit in den dortigen Arbeitsbereich des Rotors kommen. Die erfindungsgemäße Lösung vermindert ein damit einhergehendes Verletzungsrisiko auf wirksame und überraschend einfache Weise, indem der wenigstens eine Führungskanal wenigstens abschnittsweise eine Längskrümmung aufweist. Auf gesonderte Funktionsbauteile oder -komponenten zum Erreichen des Eingriffsschutzes kann somit verzichtet und ein einfacher Aufbau des Pumpengehäuses erreicht oder beibehalten werden. Zudem wird einer Wanderneigung der Fluidleitung entlang des Führungskanals und der Stützfläche unter Einwirkung des rotierenden Rotors durch die Längskrümmung entgegengewirkt. Dies ist ein zusätzlicher Vorteil. Im Übrigen erfordert die erfindungsgemäße Lösung keinerlei konstruktive oder sonstige Anpassung der Fluidleitung, wodurch zusätzlich Aufwand und Mehrkosten eingespart werden können. Zur Ausbildung des Eingriffsschutzes ist der Krümmungsradius maßlich auf die Kanallänge und den Kanaldurchmesser abgestimmt. Vorzugsweise ist der Kanaldurchmesser geringfügig größer als ein Außendurchmesser der aufzunehmenden Fluidleitung. Der Führungskanal ist durch die Gehäusewandung des Pumpengehäuses erstreckt, so dass die Kanallänge vorzugsweise durch eine Wandungsstärke der Gehäusewandung im Bereich des wenigstens einen Führungskanals konstruktiv vorgegeben ist. Dabei ist die Wandungsstärke üblicherweise im Hinblick auf eine zu erreichende mechanische Festigkeit des Pumpengehäuses dimensioniert. Vereinfacht ausgedrückt, sind die Kanallänge und der Kanaldurchmesser vorzugsweise durch übliche konstruktive Randbedingungen vorgegeben. Der Krümmungsradius ist demgegenüber im Hinblick auf die Funktion des Eingriffsschutzes dimensioniert und umso geringer, je größer die Kanallänge und/oder der Kanaldurchmesser sind. Unter "manuelles Eingreifen" ist vorzugsweise ein Eindringen mit einem Finger einer Hand zu verstehen. Weiter vorzugsweise ist der Begriff "Eingriffsschutz" vor dem Hintergrund der DIN EN 60529 (VDE 0470-1:2014-09) "Schutzarten durch Gehäuse" zu verstehen, so dass auch von einem Berührungsschutz gegen den Zugang mit einem Finger gesprochen werden kann. Der wenigstens eine Führungskanal bildet einen Ein- oder einen Auslass für die Fluidleitung. Das Pumpengehäuse kann insbesondere auch als Stator bezeichnet werden. Die Aufnahmeaussparung kann insbesondere auch als Pumpenbett bezeichnet werden. Die Stützfläche kann insbesondere auch als Lagerfläche bezeichnet werden. Die aufzunehmende Fluidleitung ist vorzugsweise eine elastisch deformierbare Schlauchleitung. Der wenigstens eine Führungskanal weist bevorzugt einen offenen Querschnitt zum vereinfachten Einbringen der Fluidleitung auf und ist hierzu beispielsweise mit einem Längsschlitz versehen. Alternativ weist der wenigstens eine Führungskanal einen geschlossenen Querschnitt auf.

In der Erfindung beträgt der Krümmungsradius maximal 50 % der Kanallänge.

Es hat sich gezeigt, dass hierdurch ein besonders wirksamer Eingriffsschutz erreicht werden kann.

In der Erfindung beträgt die Kanallänge zwischen 100 % und 200 % des Kanaldurchmessers. Dieser Wertebereich ist vorteilhaft im Hinblick auf den zu erzielenden Eingriffsschutz zum einen und zum anderen im Hinblick auf eine vorteilhafte Führung der Fluidleitung. Beträgt die Kanallänge weniger als 100 % des Kanaldurchmessers, kann unter Umständen selbst bei einer vergleichsweise starken Krümmung kein ausreichender Eingriffsschutz erreicht werden. Zudem wird durch den angegebenen Wertebereich eine zur Vermeidung der besagten Wanderneigung hinreichende Kontaktfläche und damit Reibung zwischen dem Führungskanal und der Fluidleitung erreicht. Beträgt die Kanallänge mehr als 200 % des Kanaldurchmessers, ist die Gehäusewandung im Bereich des Führungskanals vergleichsweise stark oder gar überdimensioniert. Dies ist mit einem Mehraufwand in der Fertigung und entsprechenden Mehrkosten verbunden.

In weiterer Ausgestaltung der Erfindung beträgt der Kanaldurchmesser zwischen 100 % und 105 % eines Außendurchmessers der Fluidleitung, so dass die Fluidleitung eng umschlossen in dem Führungskanal aufnehmbar ist. Dies wirkt insbesondere der Wanderneigung der Fluidleitung entgegen. Gleichzeitig wird ein Einquetschen der Fluidleitung vermieden.

In der Erfindung beträgt der Kanaldurchmesser zwischen 11 mm und 13 mm, bevorzugt 12,2 mm, die Kanallänge beträgt zwischen 11 mm und 26 mm, bevorzugt 20 mm, und der Krümmungsradius beträgt zwischen 5,5 mm und 13 mm, bevorzugt 9 mm. Die Erfinder haben erkannt, dass die vorgenannten Wertebereiche für den Kanaldurchmesser, die Kanallänge und den Krümmungsradius in ihrer Kombination besondere Vorteile bieten. Diese Vorteile sind für die bevorzugt angegebenen Werte besonders stark ausgeprägt.

In weiterer Ausgestaltung der Erfindung weist der Führungskanal einen mit dem Krümmungsradius versehenen ersten Kanalabschnitt und einen gerade erstreckten zweiten Kanalabschnitt auf. Durch die dementsprechend lediglich abschnittsweise Krümmung des Führungskanals können fertigungsseitige Vorteile erzielt werden. Beispielsweise kann der zweite Kanalabschnitt als gerade erstreckte Bohrung, Nut oder dergleichen gestaltet sein. Dies ist fertigungsseitig einfach umsetzbar. Der gekrümmte erste Kanalabschnitt kann in Bezug auf die Außenseite des Pumpengehäuses außenliegend und der zweite Kanalabschnitt kann innenliegend angeordnet sein oder umgekehrt. Der erste Kanalabschnitt und der zweite Kanalabschnitt gehen vorzugsweise tangential ineinander über.

In weiterer Ausgestaltung der Erfindung weist der erste Kanalabschnitt einends eine auf der Außenseite des Pumpengehäuses angeordnete Kanalöffnung auf und mündet andernends in den zweiten Kanalabschnitt. Dementsprechend ist der erste Kanalabschnitt bei dieser Ausgestaltung der Erfindung in Bezug auf die Außenseite außenliegend angeordnet. Der zweite Kanalabschnitt ist innenliegend angeordnet. Im Vergleich zu einer umgekehrten Anordnung der Kanalabschnitte wird der manuelle Eingriff hierdurch vergleichsweise früh unterbunden. Mit anderen Worten ausgedrückt, kann ein Anwender den Finger weniger weit in den Führungskanal hineinstecken, als dies bei einer innenliegenden Anordnung des ersten Kanalabschnitts der Fall wäre.

In weiterer Ausgestaltung der Erfindung mündet der zweite Kanalabschnitt in die Aufnahmeaussparung und geht tangential in die Stützfläche über. Hierdurch wird die Fluidleitung ausgehend von dem zweiten Kanalabschnitt kontinuierlich an die Stützfläche geführt. Hierdurch kann insbesondere eine unerwünschte Pulsation der Pumpförderung vermieden werden.

In weiterer Ausgestaltung der Erfindung nimmt der erste Kanalabschnitt zwischen 10 % und 30 % der Kanallänge ein. Dementsprechend ist der Führungskanal lediglich über eine vergleichsweise kurze Länge gekrümmt längserstreckt. Die übrige Länge nimmt vorzugsweise der gerade erstreckte zweite Kanalabschnitt ein. Durch den angegebenen Wertebereich kann insbesondere ein vorteilhafter Kompromiss zwischen einer einfachen Fertigung und dem zu erzielenden Eingriffsschutz erreicht werden.

In weiterer Ausgestaltung der Erfindung ist der Führungskanal in einer Ebene erstreckt, die parallel zu einer Rotationsebene des Rotors orientiert ist. Die Rotorebene ist senkrecht zur Rotorachse orientiert. In einem betriebsfertig an der Vorrichtung zur extrakorporalen Blutbehandlung montierten Zustand der Peristaltikpumpe ist das Pumpengehäuse vorzugsweise auf einer vertikal orientierten Gehäusefront der Vorrichtung positioniert. Die Rotorachse ist in einem solchen Zustand horizontal orientiert. Der Führungskanal ist im montierten Zustand der Peristaltikpumpe somit vorzugsweise in einer senkrecht zur Horizontalen ausgerichteten Vertikalebene erstreckt.

In weiterer Ausgestaltung der Erfindung ist der Mittelpunkt des Krümmungsradius relativ zu dem Führungskanal seitlich nach außen in Richtung einer seitlichen Außenkante des Pumpengehäuses versetzt angeordnet. Mit anderen Worten ausgedrückt, ist der Führungskanal nach außen gekrümmt. Im Vergleich zu einer nach innen, oben oder unten gerichteten Krümmung kann hierdurch insbesondere eine vorteilhafte Montierbarkeit der Peristaltikpumpe an der Vorrichtung zur extrakorporalen Blutbehandlung erreicht werden. Denn bei einer nach oben gerichteten Krümmung würde die Fluidleitung - in Bezug auf eine auf die Gehäusefront der Vorrichtung gerichtete Blickrichtung - nach vorne von der Gehäusefront abstehen. Bei einer nach unten gerichteten Krümmung müsste die Peristaltikpumpe in einem erhöhten Abstand von der Gehäusefront montiert werden, damit die Fluidleitung nicht an der Gehäusefront abknickt. Eine nach innen gerichtete Krümmung kann zu Problemen beim Anordnen von Anschlüssen zur insbesondere Druckmessung oder Zuführung von Zusatzstoffen an der Fluidleitung führen. Daher ist ein nach außen gekrümmter Führungskanal eine besonders bevorzugte Ausgestaltung der Erfindung.

In weiterer Ausgestaltung der Erfindung weist der Führungskanal einen oberseitigen Einführschlitz auf, der mittels eines Deckels abdeckbar ist. Der oberseitige Einführschlitz gestattet ein vereinfachtes Einführen der Fluidleitung in den Führungskanal. Der Einführschlitz erstreckt sich über die gesamte Länge des Führungskanals und ist ausgehend von einer Oberseite des Pumpengehäuses senkrecht zur Kanallänge durch die Gehäusewandung des Pumpengehäuses erstreckt. Vorzugsweise beträgt eine senkrecht zur Kanallänge erstreckte Schlitztiefe des Einführschlitzes zwischen 2 mm und 10 mm, bevorzugt 5,5 mm. Dies ermöglicht ein leichtes Einführen und eine nicht zu leichte Entnahme der Fluidleitung aus dem Führungskanal. Eine Schlitzbreite des Führungsschlitzes beträgt vorzugsweise zwischen 70 % bis 90 % des Kanaldurchmessers. Der Deckel dient einer Abdeckung der Oberseite des Pumpengehäuses. In einem geöffneten Zustand des Deckels ist die Aufnahmeaussparung ausgehend von der Oberseite frei zugänglich. Der Deckel ist vorzugsweise um eine Schwenkachse schwenkbeweglich an dem Pumpengehäuse angelenkt.

In weiterer Ausgestaltung der Erfindung bildet der Führungskanal einen Einlass für die Fluidleitung in das Pumpengehäuse und das Pumpengehäuse weist einen weiteren Führungskanal auf, der einen Auslass für die Fluidleitung aus dem Pumpengehäuse bildet und der in Bezug auf eine Symmetrieebene spiegelsymmetrisch zu dem Führungskanal angeordnet und gestaltet ist. Hierdurch wird ein besonders einfacher Aufbau des Pumpengehäuses erreicht. Die Rotorachse ist vorzugsweise in der Symmetrieebene erstreckt.

In weiterer Ausgestaltung der Erfindung ist die Aufnahmeaussparung in Bezug auf die Symmetrieebene spiegelsymmetrisch gestaltet. Dementsprechend ist bei dieser Ausgestaltung der Erfindung auch die Stützfläche spiegelsymmetrisch gestaltet. Hierdurch kann eine nochmals vereinfachte Gestaltung des Pumpengehäuses und somit eine besonders einfache Fertigung erreicht werden.

Die Erfindung betrifft zudem eine Vorrichtung zur extrakorporalen Blutbehandlung mit einer Peristaltikpumpe gemäß der vorstehenden Beschreibung. Die Vorrichtung ist vorzugsweise in Form eines Dialysegeräts gestaltet.

Die Erfindung betrifft zudem ein Pumpengehäuse mit den Merkmalen des Anspruchs 16. Das erfindungsgemäße Pumpengehäuse ist für eine gemäß der vorstehenden Beschreibung ausgeführte Peristaltikpumpe vorgesehen, welche einen um eine Rotorachse angetrieben rotierbaren Rotor aufweist. Das erfindungsgemäße Pumpengehäuse weist eine Aufnahmeaussparung auf, in welcher der Rotor der Peristaltikpumpe aufnehmbar ist. Die Aufnahmeaussparung weist eine bogenförmig erstreckte Stützfläche auf, welche - in Bezug auf einen in der Aufnahmeaussparung aufgenommenen Zustand des Rotors - um die Rotorachse erstreckt und radial von dem Rotor beabstandet ist. Die Stützfläche ist zum Abstützen einer radial zwischen dem Rotor und der Stützfläche in der Aufnahmeaussparung positionierbaren Fluidleitung vorgesehen ist. Die Fluidleitung ist zur Pumpförderung eines durch die Fluidleitung zu fördernden Fluids unter mechanischer Einwirkung des rotierenden Rotors abschnittsweise zwischen dem Rotor und der Stützfläche elastisch deformierbar. Das erfindungsgemäße Pumpengehäuse weist zudem wenigstens einen Führungskanal auf, der zwischen einer Außenseite des Pumpengehäuses und der Aufnahmeaussparung durch eine Gehäusewandung des Pumpengehäuses erstreckt ist und zum Führen der Fluidleitung zwischen der Außenseite und der Aufnahmeaussparung vorgesehen ist. Erfindungsgemäß ist ein durch eine wenigstens abschnittsweise längsgekrümmte Gestaltung des Führungskanals ausgebildeter Eingriffsschutz vorgesehen, wobei ein Krümmungsradius des Führungskanals derart auf eine Kanallänge und einen Kanaldurchmesser des Führungskanals abgestimmt ist, dass ein manuelles Eingreifen durch den Führungskanal in die Aufnahmeaussparung verhindert ist. Zur Vermeidung von Wiederholungen wird auf die mit der erfindungsgemäßen Peristaltikpumpe in Verbindung stehende Offenbarung verwiesen. Das zu dem Pumpengehäuse der erfindungsgemäßen Peristaltikpumpe Gesagte gilt sinngemäß im Hinblick auf das erfindungsgemäße Pumpengehäuse. Ausgestaltung des erfindungsgemäßen Pumpengehäuses weisen die Merkmale der Ansprüche 17, 18 und/oder 19 auf, deren Wortlaut zum Gegenstand der Beschreibung gemacht wird. Zu möglichen weiteren Ausgestaltungen des erfindungsgemäßen Pumpengehäuses wird zudem auf die Beschreibung der Ausgestaltungen der erfindungsgemäßen Peristaltikpumpe verwiesen.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt eine schematisch stark vereinfachte Darstellung eines Ausschnitts einer Ausgestaltung einer erfindungsgemäßen Vorrichtung zur extrakorporalen Blutbehandlung, die mit einer Ausführungsform einer erfindungsgemäßen Peristaltikpumpe versehen ist,
- Fig. 2: in schematischer Perspektivdarstellung ein Pumpengehäuse der Peristaltikpumpe, an welchem ein Deckel angelenkt ist und einen geöffneten Zustand einnimmt,
- Fig. 3: eine vergrößerte Draufsicht des Pumpengehäuses im Bereich zweier Führungskanäle und

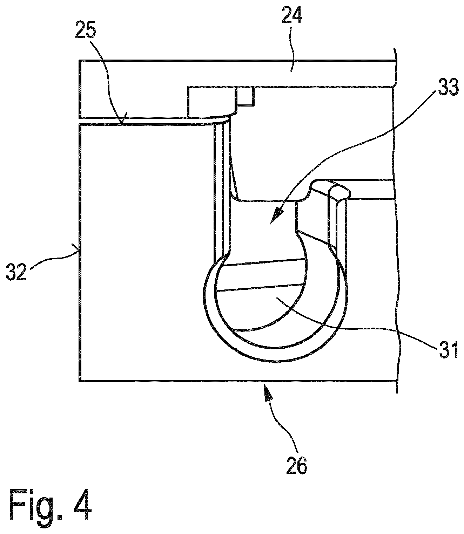

- Fig. 4: eine vergrößerte Detaildarstellung des Pumpengehäuses mit Blickrichtung in einen der Führungskanäle und in einem geschlossenen Zustand des Deckels.

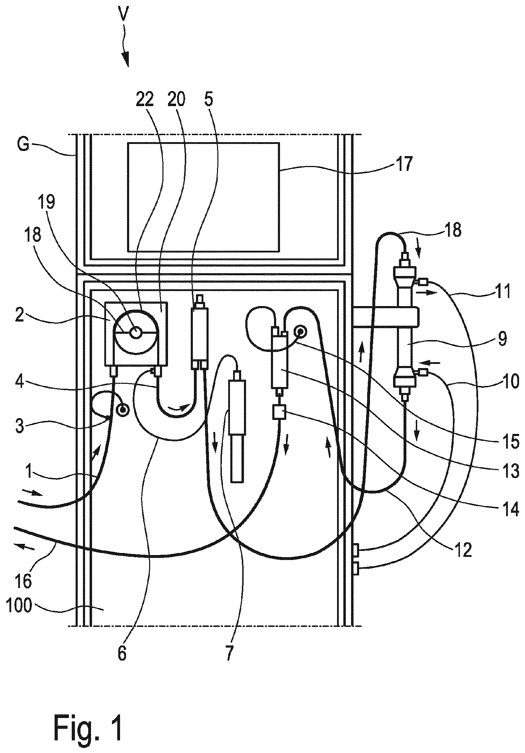

Gemäß Fig. 1 ist eine Vorrichtung V zur extrakorporalen Blutbehandlung abschnittsweise gezeigt und in Form eines Dialysegeräts gestaltet. Fig. 1 zeigt im Wesentlichen einen gesamten extrakorporalen Blutkreislauf der Vorrichtung V. Dieser weist eine arterielle Blutleitung 1 auf, mittels derer zu behandelndes Blut von einem nicht gezeigten Patienten zu einer Peristaltikpumpe 2 der Vorrichtung V geführt ist. In Bezug auf eine Förderrichtung des Bluts vor der Peristaltikpumpe 2 ist ein arterieller Druckabnehmer 3 vorgesehen. Mittels des arteriellen Druckabnehmers 3 ist der Druck in der arteriellen Blutleitung 1 vor der Peristaltikpumpe 2 erfassbar. Dieser Druck kann auch als niederseitiger Druck bezeichnet werden. In Förderrichtung des Bluts nach der Peristaltikpumpe 2 - und somit hochdruckseitig - führt eine Hochdruckblutleitung 4 zu einer arteriellen Luftfalle 5. An einem Auslass der Peristaltikpumpe 2 ist vorliegend eine Zuleitung 6 angeordnet, die an einer Pumpe 7 angeschlossen ist. Über die Zuleitung 6 kann Zusatzstoff, beispielsweise Heparin zur Blutverdünnung, zudosiert werden. Ausgehend von der arteriellen Luftfalle 5 führt eine Leitung 8 das zu behandelnde Blut zu einem Dialysator 9, welchem eingangsseitig Dialysierflüssigkeit über eine Dialysierflüssigkeitszuleitung 10 zugeführt ist. In dem Dialysator 9 wird das Blut in einer bekannten Weise mittels der Dialysierflüssigkeit behandelt. Verbrauchte Dialysierflüssigkeit, die auch als Dialysat bezeichnet werden kann, wird über eine Dialysierflüssigkeitsableitung 11 von dem Dialysator 9 abgeleitet und einer nicht dargestellten Entsorgung oder Aufbereitung zugeführt. Das behandelte Blut wird mittels einer Blutableitung 12 vom Dialysator 9 zu einer venösen Luftfalle 13 zur Abscheidung von Luft geleitet. Dieser nachgeschaltet ist ein Luftblasendetektor 14, der erkennt, ob sich für den Patienten gefährliche Luft in dem System befindet. An der venösen Luftfalle 13 ist ein venöser Druckabnehmer 15 vorgesehen, mittels dessen der venöse Druck erfassbar ist. Von der Luftfalle 13 über den Luftblasendetektor 14 wird das behandelte Blut über eine venöse Blutleitung 16 zurück zum Patienten geleitet. Zudem ist eine Steuer- und Überwachungseinrichtung 17 zur Steuerung und Überwachung der Vorrichtung V vorgesehen. Die Vorrichtung V ist in einem Gehäuse G gekapselt, das eine Gehäusefront 100 aufweist, auf welchem insbesondere die Peristaltikpumpe 2 montiert ist.

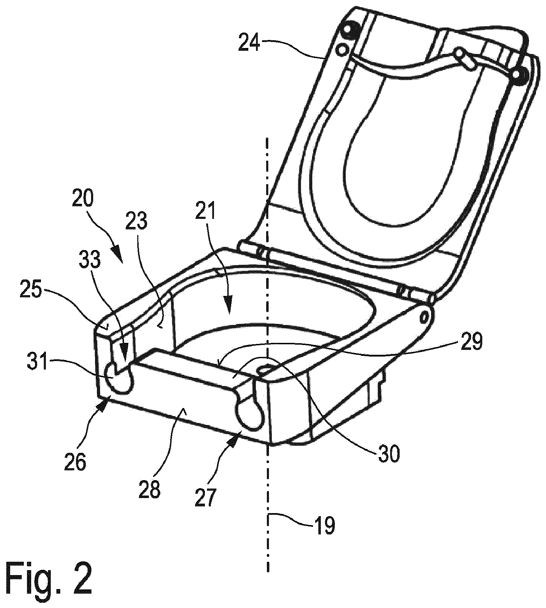

Die Peristaltikpumpe 2 weist einen Rotor 18 und ein Pumpengehäuse 20 auf, das im Detail anhand Fig. 2 gezeigt ist. Das Pumpengehäuse 20 weist eine Aufnahmeaussparung 21 auf, die auch als Pumpenbett bezeichnet werden kann. Der Rotor 18 ist um eine Rotorachse 19 angetrieben rotierbar in der Aufnahmeaussparung 21 aufgenommen. Die Aufnahmeaussparung 21 ist seitlich von einer bogenförmig um die Rotorachse 19 erstreckten und radial von dem Rotor 18 beabstandeten Stützfläche 23 begrenzt.

Zudem weist die Peristaltikpumpe 2 vorliegend einen Deckel 24 auf, der um eine nicht näher bezeichnete Schwenkachse schwenkbeweglich an dem Pumpengehäuse 20 angelenkt ist. Der Deckel 24 ist in Fig. 2 in einem geöffneten Zustand gezeigt, in welchem die Aufnahmeaussparung 21 - in Bezug auf die Zeichenebene der Fig. 2 - nach oben freigegeben ist. In einem geschlossenen Zustand deckt der Deckel 24 die Aufnahmeaussparung 21 nach oben ab und liegt hierbei auf einer Oberseite 25 des Pumpengehäuses 20 auf. In Fig. 1 ist der Deckel 24 zur besseren Darstellbarkeit des Rotors 18 und der Rotorachse 19 zeichnerisch ausgeblendet.

Die Überleitung zwischen der besagten Niederdruck- und der Hochdruckseite der Peristaltikpumpe 2 erfolgt mittels einer Fluidleitung 22 (Fig. 1), die in radialer Richtung zwischen dem Rotor 18 und der Stützfläche 23 in der Aufnahmeaussparung 21 angeordnet ist. Die Fluidleitung 22 ist bei der gezeigten Ausführungsform ein elastisch deformierbares Schlauchsegment, das an seinen gegenüberliegenden Stirnenden auf eine dem Fachmann bekannte Weise mit der arteriellen Blutleitung 1 und der Hochdruckblutleitung 4 fluidleitend verbunden ist. Zur Pumpförderung des Bluts von der Niederdruck- auf die Hochdruckseite wirkt der rotierende Rotor 18 auf die Fluidleitung 22 ein, so dass diese abschnittsweise elastisch zwischen dem Rotor 18 und der Stützfläche 23 zusammengequetscht wird. Die hierbei entstehende Abquetschung, die auch als Okklusion der Fluidleitung 22 bezeichnet werden kann, wandert gleichsam mit dem rotierenden Rotor 18 um die Rotorachse 19, so dass das Blut von der Niederdruck- auf die Hochdruckseite gefördert wird. Diese prinzipielle Funktionsweise der Peristaltikpumpe 2 ist bekannt und bedarf daher keiner weitergehenden Erörterung.

Wie weiter anhand Fig. 2 gezeigt ist, weist das Pumpengehäuse 20 vorliegend einen ersten Führungskanal 26 und einen zweiten Führungskanal 27 auf. Der erste Führungskanal 26 bildet einen Einlass für die Fluidleitung 22. Der zweite Führungskanal 27 bildet einen Auslass. Eine solche Gestaltung mit zwei Führungskanälen ist jedoch nicht als zwingend zu erachten. Bei einer zeichnerisch nicht dargestellten Ausführungsform weist das Pumpengehäuse 20 lediglich einen Führungskanal auf, der als Ein- oder Auslass fungiert, wobei ein weiterer Führungskanal beispielsweise am Deckel ausgebildet ist.

Der erste Führungskanal 26 und der zweite Führungskanal 27 sind bei der gezeigten Ausführungsform auf noch näher beschriebene Weise spiegelsymmetrisch gestaltet und angeordnet. Zur Vermeidung von Wiederholungen wird deshalb nachfolgend in erster Linie auf den ersten Führungskanal 26 Bezug genommen. Die in diesem Zusammenhang erörterten gegenständlichen und funktionellen Merkmale gelten sinngemäß für den zweiten Führungskanal 27.

Der erste Führungskanal 26, der nachfolgend kurz als Führungskanal 26 bezeichnet wird, ist zwischen einer Außenseite 28 und einer Innenseite 29 des Pumpengehäuses 20 durch eine Gehäusewandung 30 des Pumpengehäuses 20 erstreckt. Der Führungskanal 26 dient einer Aufnahme der Fluidleitung 22. Diese ist in dem anhand Fig. 1 gezeigten Gebrauchszustand der Vorrichtung V in dem Führungskanal 26 zwischen der Außenseite 28 und der Innenseite 29 geführt. Üblicherweise wird die Fluidleitung 22 zusammen mit den weiteren blutführenden Leitungsabschnitten des extrakorporalen Blutkreislaufs nach jedem Gebrauch ersetzt. In einem abgerüsteten Zustand der Vorrichtung V befindet sich dementsprechend keine Fluidleitung 22 in der Peristaltikpumpe 2 und damit auch dem Führungskanal 26. Dessen Querschnitt ist somit frei zugänglich.

Um einem unerwünschten manuellen Eingreifen durch den Führungskanal 26 in die Aufnahmeaussparung 21 entgegenzuwirken, weist das Pumpengehäuse 20 einen Eingriffsschutz auf. Der Eingriffsschutz ist durch eine wenigstens abschnittsweise längsgekrümmte Gestaltung des Führungskanals 26 ausgebildet. Hierzu ist ein Krümmungsradius R des Führungskanals 26 derart auf eine Kanallänge L und einen Kanaldurchmesser D des Führungskanals 26 abgestimmt, dass ein manuelles Eingreifen durch den Führungskanal 26 in die Aufnahmeaussparung 21 verhindert ist.

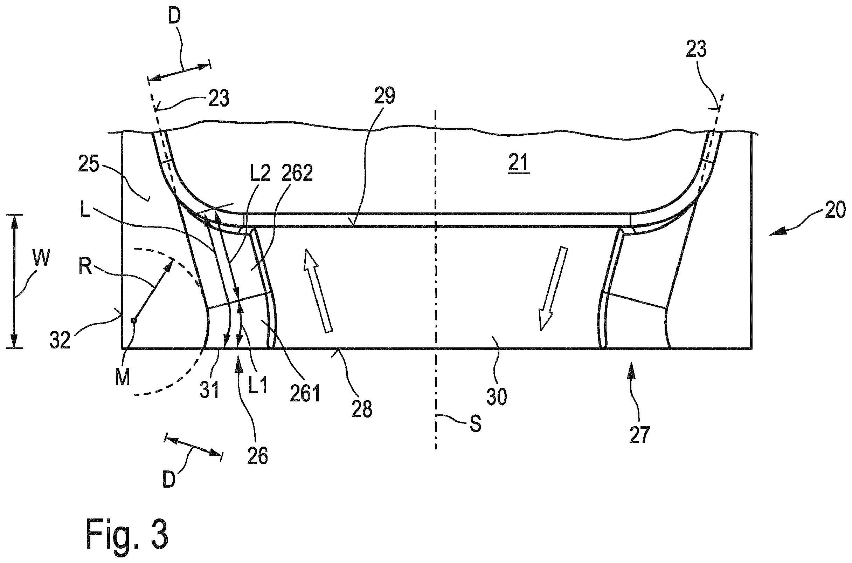

Die längsgekrümmte Gestaltung des Führungskanals 26 ist im Detail anhand Fig. 3 gezeigt. Die dort ersichtliche Gestaltung des Führungskanals 26 verhindert auf überraschend einfache Weise, dass ein Finger durch den Führungskanal 26 in die Aufnahmeaussparung 21 und damit in den Arbeitsbereich des Rotors 18 gelangen kann. Der auf diese Weise erzielte Eingriffsschutz erfordert keinerlei zusätzliche Funktionsbauteile oder -komponenten. Zudem ist keinerlei Anpassung der Fluidleitung 22 notwendig.

Der Krümmungsradius R bezieht sich vorliegend maßlich auf eine radial innenliegende Wandung des Führungskanals 26. Die Kanallänge L bezieht sich auf eine gedachte Mittellinie des Führungskanals 26.

Der Kanaldurchmesser D ist derart auf einen nicht näher bezeichneten Außendurchmesser der Fluidleitung 22 abgestimmt, dass diese eng umschlossen aber nicht etwa gequetscht in dem Führungskanal 26 geführt ist. Somit beträgt der Kanaldurchmesser D minimal 100 % des Außendurchmessers der Fluidleitung 22. Der Kanaldurchmesser D ist bei der gezeigten Ausführungsform in Abhängigkeit von den Abmessungen der aufzunehmenden Fluidleitung 22 konstruktiv vorgegeben. Die Kanallänge L ist durch die Gehäusewandung 30 erstreckt, die eine Wandungsstärke W aufweist. Näherungsweise entspricht die Kanallänge L somit der Wandungsstärke W. Jedenfalls kann die Kanallänge L die Wandungsstärke W üblicherweise nicht unterschreiten, da ansonsten keine durchgängige Verbindung zwischen der Außenseite 28 und der Innenseite 29 erreicht wäre. Die Wandungsstärke W ist insbesondere so dimensioniert, dass eine ausreichende mechanische Festigkeit des Pumpengehäuses 20 erreicht ist.

Bei der gezeigten Ausführungsform beträgt der Krümmungsradius R 50 % der Kanallänge L. Diese beträgt 200 % des Kanaldurchmessers D. Im Übrigen wird darauf hingewiesen, dass die Darstellung der Fig. 3 selbstverständlich nicht als streng maßstäblich aufzufassen ist.

Wie weiter anhand Fig. 3 gezeigt ist, weist der Führungskanal 26 vorliegend einen ersten Kanalabschnitt 261 und einen zweiten Kanalabschnitt 262 auf. Der erste Kanalabschnitt 261 ist längsgekrümmt gestaltet und weist insoweit den Krümmungsradius R auf. Demgegenüber ist der zweite Kanalabschnitt 262 gerade längserstreckt.

Der erste Kanalabschnitt 261 ist in Bezug auf die Außenseite 28 außenliegend angeordnet und weist eine auf der Außenseite 28 angeordnete Kanalöffnung 31 auf. An seinem der Kanalöffnung 31 abgewandten Stirnende geht der erste Kanalabschnitt 261 tangential in den zweiten Kanalabschnitt 262 über. Der zweite Kanalabschnitt 262 mündet an seinem dem ersten Kanalabschnitt 261 abgewandten Stirnende in die Aufnahmeaussparung 21. Dementsprechend ist auf der Innenseite 29 eine nicht näher bezeichnete Kanalöffnung vorgesehen, die einen innenliegenden Austritt des Führungskanals 26 bildet. Der zweite Kanalabschnitt 262 geht tangential in die Stützfläche 23 über.

Der erste Kanalabschnitt 261 ist bei der gezeigten Ausführungsform über eine Länge L1 erstreckt, die 30 % der Kanallänge L beträgt.

Der Führungskanal 26 ist in der Zeichenebene der Fig. 3 erstreckt. Diese ist parallel zu einer nicht näher bezeichneten und senkrecht zur Rotorachse 19 orientierten Rotationsebene des Rotors 18. Die Rotationsebene ist parallel zur Zeichenebene der Fig. 1 und damit auch zur Gehäusefront 100 des Gehäuses G orientiert. Ausgehend von einer von der Innenseite 29 in Richtung der Außenseite 28 entlang der Stützfläche 23 orientierten Blickrichtung ist der Führungskanal 26 seitlich nach außen gekrümmt. Dementsprechend ist der Mittelpunkt M des Krümmungsradius R relativ zu dem Führungskanal 26 seitlich nach außen in Richtung einer Außenkante 32 des Pumpengehäuses 20 versetzt angeordnet (Fig. 3).

Der Führungskanal 26 weist zudem einen Einführschlitz 33 auf. Dieser ist oberseitig angeordnet und senkrecht zur Längserstreckung des Führungskanals 26 durch die Gehäusewandung 30 erstreckt. Der Einführschlitz 33 erlaubt ein vereinfachtes Auf- und Abrüsten der Fluidleitung 22. Infolge des Einführschlitzes 33 bildet der Führungskanal 26 eine hinterschnittene Führungsnut, wobei der zur Aufnahme und Führung der Fluidleitung 22 vorgesehene Nutquerschnitt vorliegend kreisförmig ist.

Der zweite Führungskanal 27 ist in Bezug auf eine Symmetrieebene S (Fig. 3) spiegelsymmetrisch zu dem ersten Führungskanal 26 gestaltet und angeordnet. Die Symmetrieebene S ist in Bezug auf Fig. 1 vertikal orientiert und durch die Rotorachse 19 erstreckt. Zudem ist bei der gezeigten Ausführungsform zusätzlich die Aufnahmeaussparung 21 und damit auch die Stützfläche 23 spiegelsymmetrisch in Bezug auf die Symmetrieebene S gestaltet.

## Patentansprüche

1. Peristaltikpumpe (2) für eine Vorrichtung (V) zur extrakorporalen Blutbehandlung, aufweisend
- einen um eine Rotorachse (19) angetrieben rotierbaren Rotor (18),
- und aufweisend ein Pumpengehäuse (20) mit einer Aufnahmeaussparung (21), in welcher der Rotor (18) aufgenommen ist, und welche eine bogenförmig um die Rotorachse (19) erstreckte und radial von dem Rotor (18) beabstandete Stützfläche (23) aufweist,
- wobei die Stützfläche (23) zum Abstützen einer radial zwischen dem Rotor (18) und der Stützfläche (23) in der Aufnahmeaussparung (21) positionierbaren Fluidleitung (22) vorgesehen ist, die zur Pumpförderung eines durch die Fluidleitung (22) zu fördernden Fluids unter mechanischer Einwirkung des rotierenden Rotors (18) abschnittsweise zwischen dem Rotor (18) und der Stützfläche (23) elastisch deformierbar ist,
- und wobei das Pumpengehäuse (20) wenigstens einen Führungskanal (26) aufweist, der zwischen einer Außenseite (28) des Pumpengehäuses (20) und der Aufnahmeaussparung (21) durch eine Gehäusewandung (30) des Pumpengehäuses (20) erstreckt ist und zum Führen der Fluidleitung (22) zwischen der Außenseite (28) und der Aufnahmeaussparung (21) vorgesehen ist,
- **dadurch gekennzeichnet, dass** das Pumpengehäuse (20) einen durch eine wenigstens abschnittsweise längsgekrümmte Gestaltung des Führungskanals (26) ausgebildeten Eingriffsschutz aufweist, wobei ein Krümmungsradius (R) des Führungskanals (26) derart auf eine Kanallänge (L) und einen Kanaldurchmesser (D) des Führungskanals (26) abgestimmt ist, dass ein manuelles Eingreifen durch den Führungskanal (26) in die Aufnahmeaussparung (21) verhindert ist,
- wobei der Krümmungsradius (R) maximal 50 % der Kanallänge (L) beträgt,
- wobei die Kanallänge (L) zwischen 100 % und 200 % des Kanaldurchmessers (D) beträgt, und
- wobei der Kanaldurchmesser (D) zwischen 11 mm und 13 mm beträgt, die Kanallänge (L) zwischen 11 mm und 26 mm beträgt, und der Krümmungsradius (R) zwischen 5,5 mm und 13 mm beträgt.

2. Peristaltikpumpe (2) nach Anspruch 1, **dadurch gekennzeichnet, dass** die Fluidleitung (22) in radialer Richtung zwischen dem Rotor (18) und der Stützfläche (23) in der Aufnahmeaussparung (21) angeordnet ist und der Kanaldurchmesser (D) zwischen 100 % und 105 % eines Außendurchmessers der Fluidleitung (22) beträgt, so dass die Fluidleitung (22) eng umschlossen in dem Führungskanal (26) aufnehmbar ist.

3. Peristaltikpumpe (2) nach Anspruch 1 oder 2, **dadurch gekennzeichnet, dass** der Führungskanal (26) einen mit dem Krümmungsradius (R) versehenen ersten Kanalabschnitt (261) und einen gerade erstreckten zweiten Kanalabschnitt (262) aufweist.

4. Peristaltikpumpe (2) nach Anspruch 3, **dadurch gekennzeichnet, dass** der erste Kanalabschnitt (261) einends eine auf der Außenseite (28) des Pumpengehäuses (20) angeordnete Kanalöffnung (31) aufweist und andernends in den zweiten Kanalabschnitt (262) mündet.

5. Peristaltikpumpe (2) nach Anspruch 3 oder 4, **dadurch gekennzeichnet, dass** der zweite Kanalabschnitt (262) in die Aufnahmeaussparung (21) mündet und tangential in die Stützfläche (23) übergeht.

6. Peristaltikpumpe (2) nach einem der Ansprüche 3 bis 5, **dadurch gekennzeichnet, dass** der erste Kanalabschnitt (261) zwischen 10 % und 30 % der Kanallänge (L) einnimmt.

7. Peristaltikpumpe (2) nach einem der vorhergehenden Ansprüche, **dadurch gekennzeichnet, dass** der Führungskanal (26) in einer Ebene erstreckt ist, die parallel zu einer Rotationsebene des Rotors (18) orientiert ist.

8. Peristaltikpumpe (2) nach Anspruch 7, **dadurch gekennzeichnet, dass** der Mittelpunkt (M) des Krümmungsradius (R) relativ zu dem Führungskanal (26) seitlich nach außen in Richtung einer Außenkante (32) des Pumpengehäuses (20) versetzt angeordnet ist.

9. Peristaltikpumpe (2) nach einem der vorhergehenden Ansprüche, **dadurch gekennzeichnet, dass** der Führungskanal (26) einen oberseitigen Einführschlitz (33) aufweist, der mittels eines Deckels (24) abdeckbar ist.

10. Peristaltikpumpe (2) nach einem der vorhergehenden Ansprüche, **dadurch gekennzeichnet, dass** der Führungskanal (26) einen Einlass für die Fluidleitung (22) in das Pumpengehäuse (20) bildet, und dass das Pumpengehäuse (20) einen weiteren Führungskanal (27) aufweist, der einen Auslass für die Fluidleitung (22) aus dem Pumpengehäuse (20) bildet und der in Bezug auf eine Symmetrieebene (S) spiegelsymmetrisch zu dem Führungskanal (26) angeordnet und gestaltet ist.

11. Peristaltikpumpe (2) nach Anspruch 10, **dadurch gekennzeichnet, dass** die Aufnahmeaussparung (21) in Bezug auf die Symmetrieebene (S) spiegelsymmetrisch gestaltet ist.

12. Vorrichtung (V) zur extrakorporalen Blutbehandlung mit einer Peristaltikpumpe (2) nach einem der vorhergehenden Ansprüche.

13. Pumpengehäuse (20) für eine Peristaltikpumpe (2), aufweisend
- eine Aufnahmeaussparung (21), in welcher ein um eine Rotorachse (19) angetrieben rotierbarer Rotor (18) der Peristaltikpumpe (2) aufnehmbar ist, und welche eine bogenförmig um die Rotorachse (19) erstreckte und radial von dem Rotor (18) beabstandete Stützfläche (23) aufweist,
- wobei die Stützfläche (23) zum Abstützen einer radial zwischen dem Rotor (18) und der Stützfläche (23) in der Aufnahmeaussparung (21) positionierbaren Fluidleitung (22) vorgesehen ist, die zur Pumpförderung eines durch die Fluidleitung (22) zu fördernden Fluids unter mechanischer Einwirkung des rotierenden Rotors (18) abschnittsweise zwischen dem Rotor (18) und der Stützfläche (23) elastisch deformierbar ist,
- und aufweisend wenigstens einen Führungskanal (26), der zwischen einer Außenseite (28) des Pumpengehäuses (20) und der Aufnahmeaussparung (21) durch eine Gehäusewandung (30) des Pumpengehäuses (20) erstreckt ist und zum Führen der Fluidleitung (22) zwischen der Außenseite (28) und der Aufnahmeaussparung (21) vorgesehen ist,
- **dadurch gekennzeichnet, dass** ein durch eine wenigstens abschnittsweise längsgekrümmte Gestaltung des Führungskanals (26) ausgebildeter Eingriffsschutz vorgesehen ist, wobei ein Krümmungsradius (R) des Führungskanals (26) derart auf eine Kanallänge (L) und einen Kanaldurchmesser (D) des Führungskanals (26) abgestimmt ist, dass ein manuelles Eingreifen durch den Führungskanal (26) in die Aufnahmeaussparung (21) verhindert ist,
- wobei der Krümmungsradius (R) maximal 50 % der Kanallänge (L) beträgt,
- wobei die Kanallänge (L) zwischen 100 % und 200 % des Kanaldurchmessers (D) beträgt, und
- wobei der Kanaldurchmesser (D) zwischen 11 mm und 13 mm beträgt, die Kanallänge (L) zwischen 11 mm und 26 mm beträgt, und der Krümmungsradius (R) zwischen 5,5 mm und 13 mm beträgt.

14. Pumpengehäuse (20) nach Anspruch 13, **dadurch gekennzeichnet, dass** der Führungskanal (26) einen mit dem Krümmungsradius (R) versehenen ersten Kanalabschnitt (261) und einen gerade erstreckten zweiten Kanalabschnitt (262) aufweist.

15. Pumpengehäuse (20) nach Anspruch 14, **dadurch gekennzeichnet, dass** der erste Kanalabschnitt (261) einends eine auf der Außenseite (28) des Pumpengehäuses (20) angeordnete Kanalöffnung (31) aufweist und andernends in den zweiten Kanalabschnitt (262) mündet und/oder dass der zweite Kanalabschnitt (262) in die Aufnahmeaussparung (21) mündet und tangential in die Stützfläche (23) übergeht.

## Claims

1. Peristaltic pump (2) for a device (V) for extracorporeal blood treatment, comprising
- a rotatable rotor (18) driven around a rotor axis (19),
- and comprising a pump housing (20) with a receiving recess (21), in which the rotor (18) is received, and which has a supporting surface (23) which extends arcuately around the rotor axis (19) and is radially spaced apart from the rotor (18),
- wherein the supporting surface (23) is provided for supporting a fluid line (22) which can be positioned radially between the rotor (18) and the supporting surface (23) in the receiving recess (21) and, for pumping conveyance of a fluid to be conveyed through the fluid line (22), can be elastically deformed in sections between the rotor (18) and the supporting surface (23) under the mechanical action of the rotating rotor (18),
- and wherein the pump housing (20) has at least one guide channel (26) which extends between an outer side (28) of the pump housing (20) and the receiving recess (21) through a housing wall (30) of the pump housing (20) and is provided for guiding the fluid line (22) between the outer side (28) and the receiving recess (21),
- **characterized in that** the pump housing (20) has an intervention protection means formed by a longitudinally curved design of at least sections of the guide channel (26), wherein a radius of curvature (R) of the guide channel (26) is matched to a channel length (L) and a channel diameter (D) of the guide channel (26) in such a way that manual intervention through the guide channel (26) into the receiving recess (21) is prevented,
- wherein the radius of curvature (R) is at most 50% of the channel length (L),
- wherein the channel length (L) is between 100% and 200% of the channel diameter (D), and
- wherein the channel diameter (D) is between 11 mm and 13 mm, the channel length (L) is between 11 mm and 26 mm, and the radius of curvature (R) is between 5.5 mm and 13 mm.

2. Peristaltic pump (2) according to Claim 1, **characterized in that** that the fluid line (22) is arranged in the radial direction between the rotor (18) and the supporting surface (23) in the receiving recess (21), and the channel diameter (D) is between 100% and 105% of an external diameter of the fluid line (22), with the result that the fluid line (22) can be received in the guide channel (26) in a tightly enclosed manner.

3. Peristaltic pump (2) according to Claim 1 or 2, **characterized in that** the guide channel (26) has a first channel section (261) provided with the radius of curvature (R) and a second channel section (262) which extends in a straight line.

4. Peristaltic pump (2) according to Claim 3, **characterized in that** the first channel section (261) has, at one end, a channel opening (31) arranged on the outer side (28) of the pump housing (20) and, at the other end, opens into the second channel section (262).

5. Peristaltic pump (2) according to Claim 3 or 4, **characterized in that** the second channel section (262) opens into the receiving recess (21) and merges tangentially into the supporting surface (23).

6. Peristaltic pump (2) according to one of Claims 3 to 5, **characterized in that** the first channel section (261) occupies between 10% and 30% of the channel length (L).

7. Peristaltic pump (2) according to one of the preceding claims, **characterized in that** the guide channel (26) extends in a plane which is oriented parallel to a rotational plane of the rotor (18).

8. Peristaltic pump (2) according to Claim 7, **characterized in that** the centre point (M) of the radius of curvature (R) is arranged, relative to the guide channel (26), offset laterally outwards in the direction of an outer edge (32) of the pump housing (20).

9. Peristaltic pump (2) according to one of the preceding claims, **characterized in that** the guide channel (26) has an upper-side insertion slot (33) which can be covered by means of a cover (24).

10. Peristaltic pump (2) according to one of the preceding claims, **characterized in that** the guide channel (26) forms an inlet for the fluid line (22) into the pump housing (20), and **in that** the pump housing (20) has a further guide channel (27) which forms an outlet for the fluid line (22) from the pump housing (20) and which is arranged and designed mirror-symmetrically with respect to the guide channel (26) with regard to a plane of symmetry (S).

11. Peristaltic pump (2) according to Claim 10, **characterized in that** the receiving recess (21) is designed mirror-symmetrically with respect to the plane of symmetry (S).

12. Device (V) for extracorporeal blood treatment with a peristaltic pump (2) according to one of the preceding claims.

13. Pump housing (20) for a peristaltic pump (2), comprising
- a receiving recess (21), in which a rotatable rotor (18) of the peristaltic pump (2), driven around a rotor axis (19), can be received, and which has a supporting surface (23) which extends arcuately around the rotor axis (19) and is radially spaced apart from the rotor (18),
- wherein the supporting surface (23) is provided for supporting a fluid line (22) which can be positioned radially between the rotor (18) and the supporting surface (23) in the receiving recess (21) and, for the pumping conveyance of a fluid to be conveyed through the fluid line (22), can be elastically deformed in sections between the rotor (18) and the supporting surface (23) under the mechanical action of the rotating rotor (18),
- and comprising at least one guide channel (26) which extends between an outer side (28) of the pump housing (20) and the receiving recess (21) through a housing wall (30) of the pump housing (20), and is provided for guiding the fluid line (22) between the outer side (28) and the receiving recess (21),
- **characterized in that** an intervention protection means formed by a longitudinally curved design of at least sections of the guide channel (26) is provided, wherein a radius of curvature (R) of the guide channel (26) is matched to a channel length (L) and a channel diameter (D) of the guide channel (26) in such a way that manual intervention through the guide channel (26) into the receiving recess (21) is prevented,
- wherein the radius of curvature (R) is at most 50% of the channel length (L),
- wherein the channel length (L) is between 100% and 200% of the channel diameter (D), and
- wherein the channel diameter (D) is between 11 mm and 13 mm, the channel length (L) is between 11 mm and 26 mm, and the radius of curvature (R) is between 5.5 mm and 13 mm.

14. Pump housing (20) according to Claim 13, **characterized in that** the guide channel (26) has a first channel section (261) provided with the radius of curvature (R) and a second channel section (262) which extends in a straight line.

15. Pump housing (20) according to Claim 14, **characterized in that** the first channel section (261) has, at one end, a channel opening (31) arranged on the outer side (28) of the pump housing (20) and, at the other end, opens into the second channel section (262), and/or **in that** the second channel section (262) opens into the receiving recess (21) and merges tangentially into the supporting surface (23).

## Revendications

1. Pompe péristaltique (2) pour un dispositif (V) de traitement de sang extracorporel, présentant
- un rotor (18) rotatif entraîné autour d'un axe de rotor (19),
- et présentant un corps de pompe (20) avec un évidement de réception (21), dans lequel le rotor (18) est reçu, et qui présente une surface de soutien (23) s'étendant en forme d'arc autour de l'axe de rotor (19) et espacée radialement du rotor (18),
- la surface de soutien (23) étant prévue pour soutenir une conduite de fluide (22) pouvant être positionnée radialement entre le rotor (18) et la surface de soutien (23) dans l'évidement de réception (21), qui peut être déformée élastiquement par sections entre le rotor (18) et la surface de soutien (23) pour le transport par pompage d'un fluide à transporter par la conduite de fluide (22) sous l'action mécanique du rotor (18) en rotation,
- et le corps de pompe (20) présentant au moins un canal de guidage (26) qui s'étend entre un côté extérieur (28) du corps de pompe (20) et l'évidement de réception (21) à travers une paroi de corps (30) du corps de pompe (20) et est prévu pour guider la conduite de fluide (22) entre le côté extérieur (28) et l'évidement de réception (21),
- **caractérisé en ce que** le corps de pompe (20) présente une protection contre l'engagement réalisée par une configuration courbée longitudinalement au moins par sections du canal de guidage (26), un rayon de courbure (R) du canal de guidage (26) étant adapté à une longueur de canal (L) et à un diamètre de canal (D) du canal de guidage (26) de telle sorte qu'un engagement manuel à travers le canal de guidage (26) dans l'évidement de réception (21) est empêché,
- le rayon de courbure (R) représentant au maximum 50 % de la longueur de canal (L),
- la longueur de canal (L) étant comprise entre 100 % et 200 % du diamètre de canal (D), et
- le diamètre de canal (D) étant compris entre 11 mm et 13 mm, la longueur de canal (L) étant comprise entre 11 mm et 26 mm, et le rayon de courbure (R) étant compris entre 5,5 mm et 13 mm.

2. Pompe péristaltique (2) selon la revendication 1, **caractérisée en ce que** la conduite de fluide (22) est agencée dans la direction radiale entre le rotor (18) et la surface de soutien (23) dans l'évidement de réception (21) et le diamètre de canal (D) est compris entre 100 % et 105 % d'un diamètre extérieur de la conduite de fluide (22), de telle sorte que la conduite de fluide (22) peut être reçue de manière étroitement enfermée dans le canal de guidage (26).

3. Pompe péristaltique (2) selon la revendication 1 ou 2, **caractérisée en ce que** le canal de guidage (26) comprend une première section de canal (261) pourvue du rayon de courbure (R) et une deuxième section de canal (262) s'étendant en ligne droite.

4. Pompe péristaltique (2) selon la revendication 3, **caractérisée en ce que** la première section de canal (261) présente à une extrémité une ouverture de canal (31) agencée sur le côté extérieur (28) du corps de pompe (20) et débouche à l'autre extrémité dans la deuxième section de canal (262).

5. Pompe péristaltique (2) selon la revendication 3 ou 4, **caractérisée en ce que** la deuxième section de canal (262) débouche dans l'évidement de réception (21) et se raccorde tangentiellement à la surface de soutien (23).

6. Pompe péristaltique (2) selon l'une quelconque des revendications 3 à 5, **caractérisée en ce que** la première section de canal (261) occupe entre 10 % et 30 % de la longueur de canal (L).

7. Pompe péristaltique (2) selon l'une quelconque des revendications précédentes, **caractérisée en ce que** le canal de guidage (26) s'étend dans un plan orienté parallèlement à un plan de rotation du rotor (18).

8. Pompe péristaltique (2) selon la revendication 7, **caractérisée en ce que** le centre (M) du rayon de courbure (R) est agencé décalé latéralement vers l'extérieur par rapport au canal de guidage (26) en direction d'un bord extérieur (32) du corps de pompe (20).

9. Pompe péristaltique (2) selon l'une quelconque des revendications précédentes, **caractérisée en ce que** le canal de guidage (26) présente une fente d'introduction (33) du côté supérieur, qui peut être recouverte au moyen d'un couvercle (24).

10. Pompe péristaltique (2) selon l'une quelconque des revendications précédentes, **caractérisée en ce que** le canal de guidage (26) forme une entrée pour la conduite de fluide (22) dans le corps de pompe (20), et **en ce que** le corps de pompe (20) présente un autre canal de guidage (27) qui forme une sortie pour la conduite de fluide (22) hors du corps de pompe (20) et qui est agencé et configuré avec une symétrie spéculaire par rapport à un plan de symétrie (S) par rapport au canal de guidage (26).

11. Pompe péristaltique (2) selon la revendication 10, **caractérisée en ce que** l'évidement de réception (21) est configuré avec une symétrie spéculaire par rapport au plan de symétrie (S).

12. Dispositif (V) de traitement de sang extracorporel, avec une pompe péristaltique (2) selon l'une quelconque des revendications précédentes.

13. Corps de pompe (20) pour une pompe péristaltique (2), présentant
- un évidement de réception (21), dans lequel peut être reçu un rotor (18) de la pompe péristaltique (2) pouvant être entraîné en rotation autour d'un axe de rotor (19), et qui présente une surface de soutien (23) s'étendant en forme d'arc autour de l'axe de rotor (19) et espacée radialement du rotor (18),
- la surface de soutien (23) étant prévue pour soutenir une conduite de fluide (22) pouvant être positionnée radialement entre le rotor (18) et la surface de soutien (23) dans l'évidement de réception (21), qui peut être déformée élastiquement par sections entre le rotor (18) et la surface de soutien (23) pour le transport par pompage d'un fluide à transporter par la conduite de fluide (22) sous l'action mécanique du rotor (18) en rotation,
- et présentant au moins un canal de guidage (26) qui s'étend entre un côté extérieur (28) du corps de pompe (20) et l'évidement de réception (21) à travers une paroi de corps (30) du corps de pompe (20) et est prévu pour guider la conduite de fluide (22) entre le côté extérieur (28) et l'évidement de réception (21),
- **caractérisé en ce qu'**une protection contre l'engagement réalisée par une configuration courbée longitudinalement au moins par sections du canal de guidage (26) est prévue, un rayon de courbure (R) du canal de guidage (26) étant adapté à une longueur de canal (L) et à un diamètre de canal (D) du canal de guidage (26) de telle sorte qu'un engagement manuel à travers le canal de guidage (26) dans l'évidement de réception (21) est empêché,
- le rayon de courbure (R) représentant au maximum 50 % de la longueur de canal (L),
- la longueur de canal (L) étant comprise entre 100 % et 200 % du diamètre de canal (D), et
- le diamètre de canal (D) étant compris entre 11 mm et 13 mm, la longueur de canal (L) étant comprise entre 11 mm et 26 mm, et le rayon de courbure (R) étant compris entre 5,5 mm et 13 mm.

14. Corps de pompe (20) selon la revendication 13, **caractérisée en ce que** le canal de guidage (26) présente une première section de canal (261) pourvue du rayon de courbure (R) et une deuxième section de canal (262) s'étendant en ligne droite.

15. Corps de pompe (20) selon la revendication 14, **caractérisé en ce que** la première section de canal (261) présente à une extrémité une ouverture de canal (31) agencée sur le côté extérieur (28) du corps de pompe (20) et débouche à l'autre extrémité dans la deuxième section de canal (262) et/ou **en ce que** la deuxième section de canal (262) débouche dans l'évidement de réception (21) et se raccorde tangentiellement à la surface de soutien (23).
